# EUROPEAN PATENT SPECIFICATION

(11) **EP 3 256 572 B1**
(45) Date of publication and mention of the grant of the patent: **21.08.2019**
(21) Application number: 16704539.2
(22) Date of filing: 05.02.2016
(51) Int. Cl.: G01N 33/569, G01N 33/574, B01L 3/00, C12N 7/02

(54) **IMMOBILIZATION OF CELLS OR VIRUS PARTICLES ON PROTEIN STRUCTURES USING A MICROFLUIDIC CHAMBER**
IMMOBILISIERUNG VON ZELLEN- ODER VIRUSTEILCHEN AUF PROTEINSTRUKTUREN MIT EINER MIKROFLUIDISCHEN KAMMER
IMMOBILISATION DE CELLULES OU DE PARTICULES DE VIRUS SUR DES STRUCTURES PROTÉIQUES AU MOYEN D'UNE CHAMBRE MICROFLUIDIQUE

(30) Priority: 13.02.2015 EP 15000479
(43) Date of publication of application: 20.12.2017
(73) Proprietor: Karlsruher Institut für Technologie (KIT), 76131 Karlsruhe (DE); Universitätsklinikum Hamburg-Eppendorf (UKE), 20246 Hamburg (DE)
(72) Inventor: HIRTZ, Michael, 76351 Linkenheim-Hochstetten (DE); FUCHS, Harald, 48145 Münster (DE); PANTEL, Klaus, 22457 Hamburg (DE); BRINKMANN, Falko, 22081 Hamburg (DE)
(74) Representative: Müller-Boré & Partner Patentanwälte PartG mbB
(86) International application number: PCT/EP2016/000191
(87) International publication number: WO 2016/128125

(56) References cited:
- WO-A1-2014/197455
- WO-A2-2007/106598
- WO-A2-2010/124227
- US-A1- 2009 311 668
- US-A1- 2012 100 538
- US-A1- 2014 146 636
- MARY NORA DICKSON ET AL: "Efficient capture of circulating tumor cells with a novel immunocytochemical microfluidic device", BIOMICROFLUIDICS, vol. 5, no. 3, 22 August 2011 (2011-08-22) , pages 34119-1, XP055034319, US ISSN: 1932-1058, DOI: 10.1063/1.3623748]
- TAKUMA HORII ET AL: "Rapid formation of cell-particle complexes via dielectrophoretic manipulation for the detection of surface antigens", BIOSENSORS AND BIOELECTRONICS, vol. 61, 21 May 2014 (2014-05-21), pages 215-221, XP055202692, ISSN: 0956-5663, DOI: 10.1016/j.bios.2014.05.019
- JENNIFER O. FOLEY ET AL: "Experimental and model investigation of the time-dependent 2-dimensional distribution of binding in a herringbone microchannel", LAB ON A CHIP, vol. 8, no. 4, 1 April 2008 (2008-04-01), pages 557-564, XP055019078, ISSN: 1473-0197, DOI: 10.1039/b713644g
- LIH FENG CHEOW ET AL: "Continuous-flow biomolecule concentration and detection in a slanted nanofilter array", LAB ON A CHIP, vol. 12, no. 21, 1 January 2012 (2012-01-01), page 4441, XP055221932, GB ISSN: 1473-0197, DOI: 10.1039/c2lc40195a
- S. L. STOTT ET AL: "Isolation of circulating tumor cells using a microvortex-generating herringbone-chip", PROCEEDINGS OF THE NATIONAL ACADEMY OF SCIENCES, vol. 107, no. 43, 26 October 2010 (2010-10-26), pages 18392-18397, XP055113098, ISSN: 0027-8424, DOI: 10.1073/pnas.1012539107

## Description

The present invention relates to methods for the immobilization of cells or virus particles of interest expressing one or more predetermined surface marker(s) on defined spots on a solid support, comprising the steps of providing a biological fluid sample suspected of containing cells or virus particles of interest; labeling the cells or virus particles of interest with antibodies, antibody fragments or antibody mimetics directed against the one or more predetermined surface marker(s) and carrying a first binding agent; and contacting the labeled cells or virus particles of interest with a solid support (biochip), said solid support comprising an array of defined isolated spots of a solid support-bound second binding agent, wherein the first and second binding agents can bind to each other. The present invention further relates to devices for the isolation of cells or virus particles of interest expressing one or more predetermined surface marker(s), comprising a microfluidic chamber comprising a solid support comprising an array of defined isolated spots of a solid support-bound binding agent, and a surface opposing the solid support, said surface having transverse structures that facilitate chaotic mixing of the fluid containing the cells or virus particles of interest when said fluid flows along said surface; and a capillary micropipette.

The origin of metastases in cancer are circulating tumor cells (CTCs). These cells separate from the solid primary tumor and enter the blood stream. While circulating in their new environment, CTCs can settle in organs and undergo mitosis. Avoiding and controlling metastases is crucial for cancer treatment, since these metastases are most of the times responsible for the patient's ultimate fate, while the primary tumor seldom leads to life threatening conditions on itself. However, CTCs offer new perspectives in the observation and treatment of cancer. Known as liquid biopsy, frequent quantification and molecular analysis of CTCs before and during treatment supply a real-time status of the patient, rather than the fixed one-time histopathologic result of the primary tumor.

The identification and hence enrichment and localization of cells of interest, such as CTCs, but also other cells including bacterial cells, or virus particles of interest in biological fluids is still a challenge, even after decades of research. This is particularly true for cells or virus particles that are present in the biological fluid in very low numbers, possibly on a background of high numbers of other cells or virus particles. For example, in blood the ratio between CTCs and blood cells is approximately 1:10⁹ (assuming < 200 CTC/ml, 5x10⁹ red blood cells/ml, 7x10⁶ white blood cells/ml).

Various strategies for the enrichment and identification of cells in biological fluids have been described. Physical or biological characteristics of cells can be exploited to enrich their concentration in a given biological fluid. Filtration concerning cell size or stiffness can be performed as well as a density centrifugation, where particular cells can be isolated. As another target for enrichment and separation, specific surface markers can be facilitated in several ways for capturing. Binding agents binding to such surface markers conjugated with magnetic nanoparticles mark cells that separate from non-labeled cells in a magnetic field. The same principle can be performed by negative enrichment or depletion. Further, most microfluidic approaches are based on the immobilization of cells surfaces labeled with specific binding agents.

After enrichment according to methods known in the art, cells still need to be identified in an additional step. Cytospinning is a technique that centrifuges cells from suspension onto a surface. Immunostaining approaches can then be performed to mark proteins that are either specific to the cells of interest or identify said cells by exclusion of other cell types. Systems like CellSearch perform enrichment and identification in a fully automated way. Furthermore, automated imaging systems based on fluorescence microscopy observe cytospinned cell samples and supply micrographs of single cells. Genomic analyses of cell suspensions or even single cells allow the precise characterization and absolute identification of cells of interest, *e.g.* via procedures like the polymerase chain reaction and methods like whole genome sequencing. Besides exploitation of biological properties for enrichment, chip technologies also facilitate physical parameters for cell enrichment. Even both the biological and physical approach can be conducted in one chip serially.

Since the discovery of the epithelial cell adhesion molecule (EpCAM) as a marker for CTCs, various microfluidic approaches were developed showing promising results. Anti-EpCAM coated surfaces interact with EpCAM molecules in the cell membrane immobilizing the CTCs whereas blood cells transmit the system. Verification and further analysis is carried out by immunostaining. However, recent studies have shown that EpCAM is not always a reliable marker. Some patients develop metastases although EpCAM positive CTCs are not detectable in their blood. Further, CTCs were discovered that were negative for anti-EpCAM staining. On this account, the development of CTC-capturing devices that can easily target different surface epitomes besides EpCAM, are able to handle high blood volumes and allow single cell analysis is still challenging, but highly demanded.

In this context, Dickson *et al.* (Dickson, M. N. et al.; Biomicrofluidics, 5(3); 2011; pages 34119-1 to 34119-15) describe means for the capture of circulating tumor cells (CTCs) in an immunocytochemical device. Scott et al. (Scott SL et al., PNAS,107(43); 2010; pages 18392-97) describe the isolation of CTCs in a microvortex-generating herringbone chip using antibody-coated microposts. Further, US 2012/0100538 A1 describes a microfluidic device for isolating target biomolecules or cells from samples, as well as methods of using the same. Furthermore, WO 2014/197455 A1 describes microfluidic devices and methods of isolating target cells from a population of cells.

Respective methods known in the art all require the use of labeled, e.g. fluorescently labeled, specific binding agents for identification of the isolated cells after immobilization. Further, said methods often achieve only inadequate binding efficiency, specificity and recovery of cells.

Accordingly, the technical problem underlying the present invention is to provide means for the immobilization, identification and isolation of cells or virus particles of interest from biological fluids, in particular cells or virus particles that are present in the biological fluid in very low numbers, possibly on a background of high numbers of other cells or virus particles. Such means should display a high binding efficiency, high specificity and high recovery, and enable the direct identification of the cells or virus particles of interest without the use of labeled specific binding agents. Further, such means should allow for an easy access to immobilized cells for subsequent single cell analysis.

The solution to the above technical problem is achieved by the embodiments characterized in the claims.

In particular, in a first aspect, the present invention relates to a method for the immobilization of cells or virus particles of interest expressing one or more predetermined surface marker(s) on defined spots on a solid support, comprising the steps:
(a) providing a biological fluid sample suspected of containing cells or virus particles of interest;
(b) labeling the cells or virus particles of interest with antibodies, antibody fragments or antibody mimetics directed against the one or more predetermined surface marker(s) and carrying a first binding agent; and
(c) contacting the labeled cells or virus particles of interest with a solid support, said solid support comprising an array of defined isolated spots of a solid support-bound second binding agent, wherein the first and second binding agents can bind to each other, and wherein the solid support is part of a microfluidic chamber, said microfluidic chamber comprising:
   (i) said solid support; and
   (ii) a surface opposing the solid support, said surface having transverse structures that facilitate chaotic mixing of the fluid containing the cells or virus particles of interest when said fluid flows along said surface, wherein said transverse structures are surface ridges having a herringbone-structure.

In the present application, the term "comprising" is expressly intended to encompass the terms "consisting of" and "consisting essentially of". Thus, said terms are interchangeable throughout the present application.

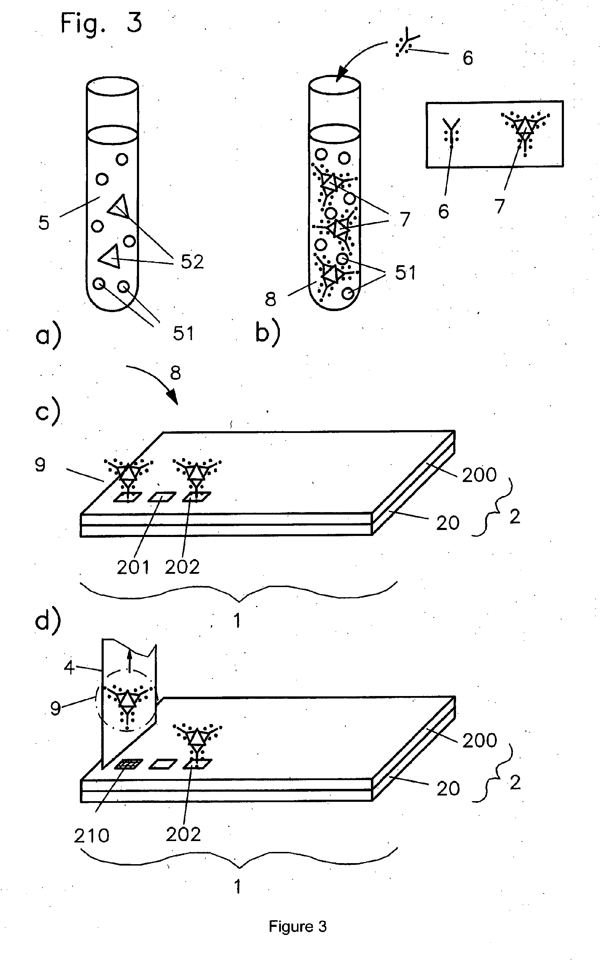

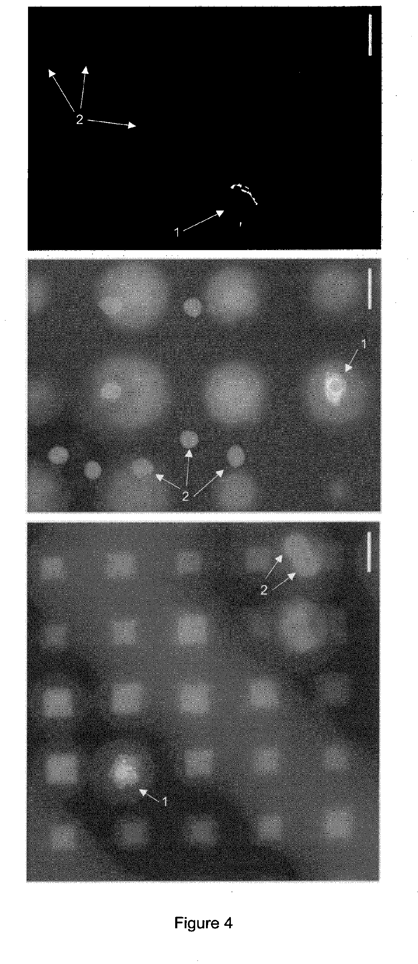

The method of the present invention can be used for the immobilization of particular cells of interest, or of particular virus particles of interest, provided that said cells or virus particles express a known surface marker, and provided that antibodies, antibody fragments or antibody mimetics that bind to said known surface marker in a specific manner are available. Figure 3 provides a schematic overview of the method of the present invention, whereas Figure 4 shows fluorescently labeled cells immobilized on a solid support in accordance with said method. In this context, antibody fragments are not particularly limited and are known in the art. They include Fab fragments, F(ab')₂ fragments and Fab' fragments. Further, antibody mimetics are not particularly limited and are known in the art. They include single-chain variable fragment (scFv) antibodies, affibodies, affilins, affimers, affitins, anticalins, nucleic acid aptamers, peptide aptamers, avimers, DARPins, Kunitz domain peptides, and monobodies.

Preferably, the method of the present invention is used for the immobilization of cells, wherein cells that are present in the biological fluid in very low numbers, possibly on a background of high numbers of other cells, are particularly preferred. In a specific embodiment, the cells are circulating tumor cells (CTCs).

Surface markers that can function as a labeling target in the context of the method of the present invention are not particularly limited, provided that antibodies, antibody fragments or antibody mimetics that bind to said known surface marker in a specific manner are available. In a specific embodiment, the surface marker is Epithelial Cell Adhesion Molecule (EpCAM) which is an important marker of CTCs. Further particular surface markers that can be of interest include human epidermal growth factor receptor 2 (HER-2) which is a marker of breast cancer cells, CD44, CD133, aldehyde dehydrogenase isoform 1 (ALDH1), c-Met, CD47, nerve-cell adhesion molecule (N-CAM), CD340, epidermal growth factor receptor (EGFR), CD318, Muc-1, Trop-2, Mpv-18, and musculin (MSC).

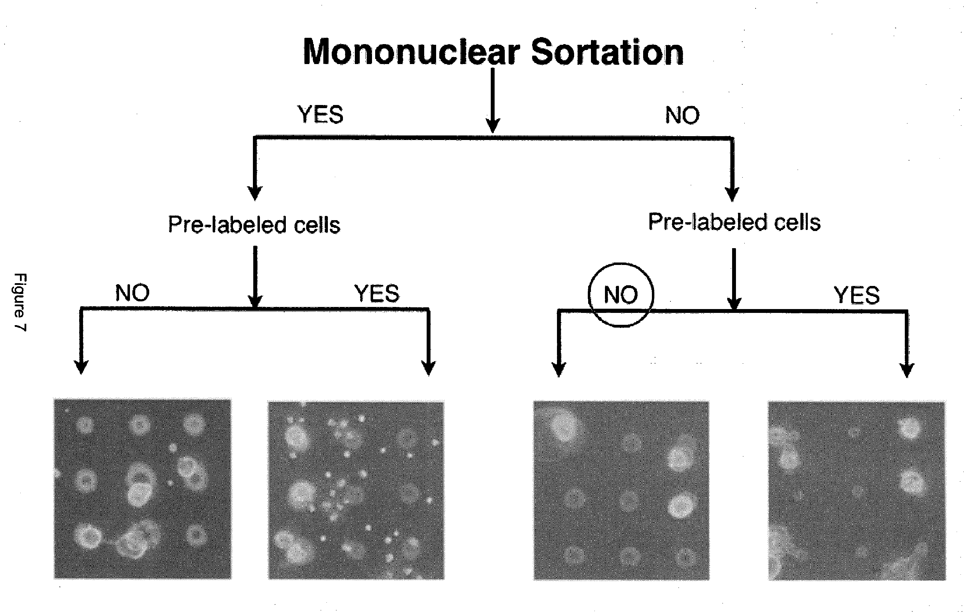

The biological fluid provided in the method of the present invention is a biological fluid that is suspected of containing cells or virus particles of interest. Respective biological fluids are not particularly limited. They include for example biological fluids selected from the group consisting of blood, blood components, amniotic fluid, cerebrospinal fluid, lymphatic fluid, peritoneal fluid, saliva, sputum, urine, and partitions or fractions thereof. In this context, the method of the present invention may, in a preferred embodiment, comprise after step (a) and prior to step (b), or after step (b) and prior to step (c), the step of enriching cells or virus particles of interest from the biological fluid. As an example, in case the biological fluid is blood, mononuclear cells may be enriched by way of a density gradient centrifugation as known in the art. Further means for enriching cells or virus particles of interest from a biological fluid are not particularly limited and are known in the art. Figure 7 shows data from an experiment wherein either whole blood or only leukocytes enriched therefrom have been incubated on a coverslip carrying an array according to the present invention, and MCF-7 cells have been either labeled before spiking into the blood cell suspension or labeled in the blood cell suspension.

In step (b) of the method of the present invention, cells or virus particles of interest are labeled with antibodies, antibody fragments or antibody mimetics as defined above which are directed against the one or more predetermined surface marker(s), *i.e*., which bind in a specific mannerto said surface markers, wherein said antibodies, antibody fragments or antibody mimetics carry a first binding agent. In this context, the term "carrying a first binding agent" as used herein indicates that the antibodies, antibody fragments or antibody mimetics are non-covalently or, preferably, covalently coupled to said first binding agent. Respective binding agents and binding agent pairings of first and second binding agents that can bind to each other are not particularly limited and are known in the art. In a preferred embodiment, the first binding agent is biotin. In another preferred embodiment, the first binding agent is a His-Tag.

In step (c) of the method of the present invention, the labeled cells or virus particles of interest are contacted with a solid support, said solid support comprising an array of defined isolated spots of a solid support-bound second binding agent, wherein the first and second binding agents can bind to each other.

The second binding agent that is bound to the solid support is not particularly limited, provided that it can bind to the first binding agent carried by the antibodies, antibody fragments or antibody mimetics. As indicated above, respective pairings of first and second binding agents that can bind to each other are not particularly limited and are known in the art. In a preferred embodiment, the first binding agent is biotin and the second binding agent is streptavidin or an analog thereof such as avidin and neutravidin. In another preferred embodiment, the first binding agent is a His-Tag and the second binding agent is a metal chelating group, e.g. nitrilotriacetic acid (NTA). Further, in a preferred embodiment, the second binding agent is fluorescently labeled, wherein suitable fluorescent dyes are not particularly limited and can be chosen from fluorescent dyes known in the art. In a particular example, said fluorescent dye is the cyanine dye Cy3.

The solid support that the cells or virus particles are contacted with is a solid substrate, preferably a transparent solid substrate. Suitable materials are not particularly limited and are known in the art. In a preferred embodiment, the solid support is a glass substrate. In other embodiments, the solid support is a substrate made of a transparent polymer material such as e.g. polystyrene and poly(methyl methacrylate). Said solid support is in some instances designated as "biochip" throughout the present application. Preferably, the solid support provides a plane, smooth surface.

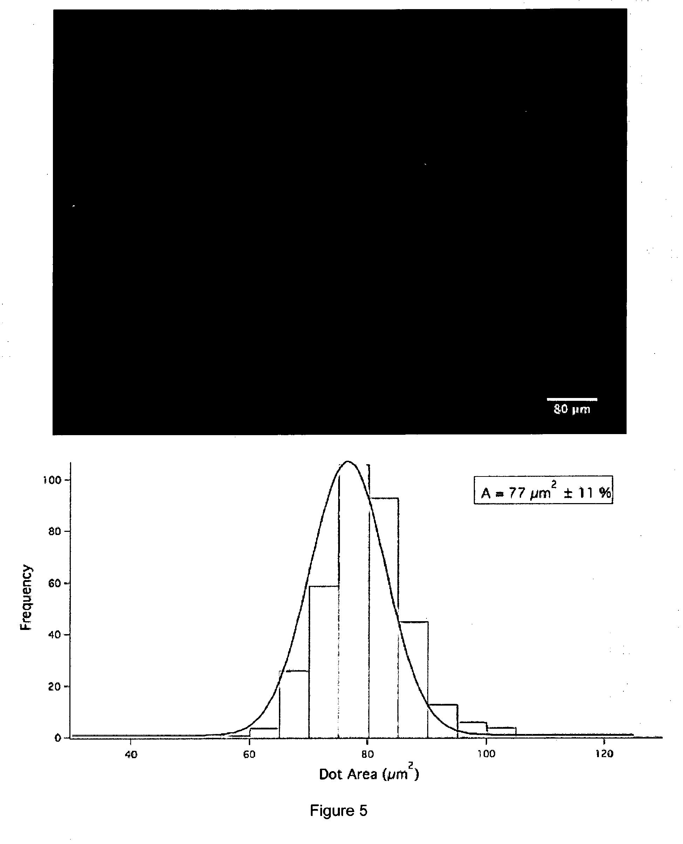

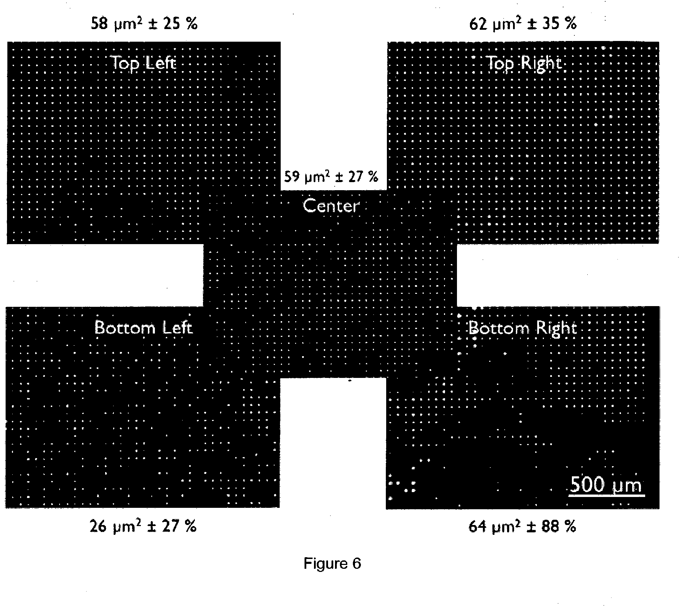

The solid support used in the method of the present invention comprises an array of defined isolated spots of solid support-bound second binding agent. This array is preferably a regular array of spots, the array covering an area of 1 to 2500 mm², preferably 25 to 1000 mm², more preferably of 100 to 600 mm², most preferably of 300 to 500 mm², e.g. of 400 mm², wherein the dots are spaced at a distance of at least 10 µm, preferably at least 15 µm, more preferably at least 20 µm, e.g. 20 to 100 µm, 20 to 80 µm, 20 to 60 µm, 20 to 40 µm, 20 to 30 µm, or 25 µm. The spot diameter is preferably 2 to 20 µm, more preferably 5 to 15 µm, more preferably 8 to 12 µm, e.g. 10 µm. Respective arrays are shown in Figures 5 and 6. The term "defined isolated spots" as used herein indicates that the spots have a sharp and clearly defined boundary and are isolated from each other by areas that do not have solid support-bound second binding agent.

Methods for generating arrays of defined isolated spots of binding agents according to the present invention on a solid support are not particularly limited and are known in the art. They include methods involving the direct application of the binding agents or the application of anchoring molecules for subsequent binding of the binding agents to said anchoring molecules by dip-pen nanolithography (DPN), polymer pen lithography (PPL), spotting with capillaries, microchannel cantilevers or ink jet printing to the solid support. This may involve prior activation of the solid support by means known in the art. Specific exemplary and preferred methods include the methods described hereinafter. In a first example, the solid support is passivated with bovine serum albumin (BSA) by immersing the solid support in a solution of BSA. Subsequently, an array of biotin-4-fluorescein is printed on the solid support by PPL. Irradiation of the solid support with UV light covalently couples the biotin-4-fluorescein to the BSA on the solid support (cf. Examples, Experimental procedures). After washing, the solid support is incubated with streptavidin which binds to the immobilized biotin, thus generating an array of solid support-bound streptavidin. In a second example, the strong affinity of thiol groups (-SH) to gold is used. The solid support is coated with gold and subsequently an array of Thiol-biotin is printed by PPL or DPN on the solid support, followed by incubation with streptavidin (cf. Example 4). In a third example, cycloaddition of azide groups to alkyne groups is used. The solid support is e.g. immersed in a solution of (3-glycidyloxypropyl)trimethoxysilane (GPTMS) and an epoxy ring-opening is performed by immersing the solid support in a solution of propargylamine. Subsequently, an array of biotin-azide is printed on the solid support by DPN, followed by incubation with streptavidin (cf. Example 4). In related embodiments, biotin-alkyne is printed on a solid support having immobilized azide groups.

In preferred embodiments, the areas on the solid support surrounding the defined isolated spots of solid support-bound second binding agent are passivated. Suitable methods for the passivation of surfaces are not particularly limited and are known in the art. They include for examples the incubation of the surface with solutions of one or more suitable passivation agents. Respective passivation agents are not particularly limited and are known in the art. They include for example BSA, milk powder, casein, and polyethylene glycol (PEG). In this context, the term "passivation" as used herein indicates the process of rendering a surface unable to unspecifically bind or adhere proteins, cells, or virus particles.

According to the present invention, the solid support is part of a microfluidic chamber, said microfluidic chamber comprising said solid support, preferably as the bottom surface of the chamber, and a surface opposing the solid support, preferably as the ceiling surface of the chamber, said surface opposing the solid support having transverse structures that facilitate chaotic mixing of the fluid containing the cells or virus particles of interest when said fluid flows along said surface. The microfluidic chamber can further comprise inlet and outlet ports for supplying and discharging the biological fluid to and from the chamber, respectively. Preferably, all surfaces of the chamber are passivated in addition to the solid support, as specified above.

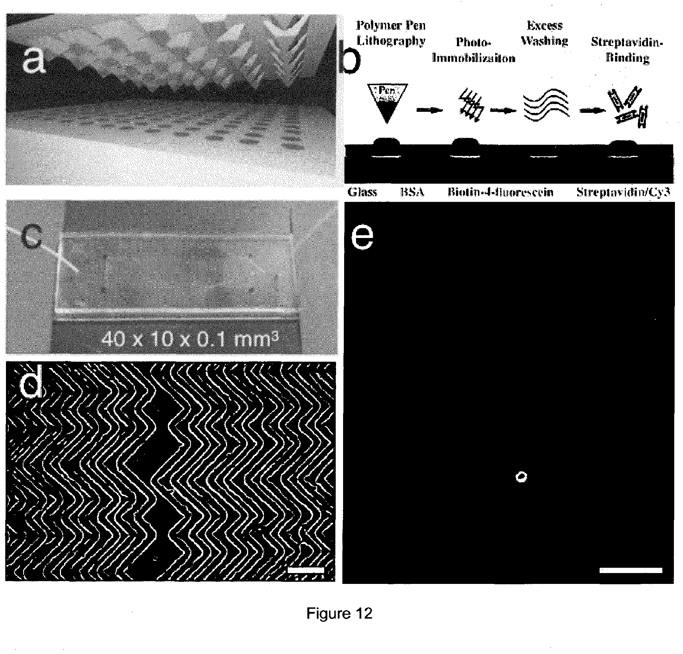

According to the present invention, said transverse structures are surface ridges having a herringbone-structure as known in the art and as depicted in Figure 12d. Respective transverse structures increase the interaction of cells or virus particles with the solid support by causing a stirring effect in the biological fluid and, hence, increase the probability of contact to the solid support and distribute the cells homogeneously over the whole array.

In preferred embodiments, the microfluidic chamber has a channel height of 10 to 150 µm, preferably 20 to 100 µm, more preferably 30 to 70 µm, or 40 to 60 µm, e.g. 50 µm. as measured from the solid support to the surface ridges. The surface ridges have preferably a height of 20 to 70 µm, preferably, 30 to 60 µm, more preferably 40 to 50 µm, e.g. 45 µm. Accordingly, the height from the solid support to the surface opposing the solid support in-between two surface ridges can be from 30 to 220 µm, e.g. from 80 to 110 µm, or from 85 to 95 µm.

Further, regarding the above herringbone-structure, two consecutive ridges of said structure can have a distance from each other as measured in the flow direction of 20 to 80 µm, preferably 30 to 70 µm, or 40 to 60 µm, e.g. 50 µm. A single ridge can be composed of alternating segments that are arranged to alternately form an angle with the flow direction of 45°, then 135°, then 45° and so on, *i.e*., said segments form a 90° angle with each other. Two consecutive segments can have a shorter length, *e.g.* of 120 to 160 µm, 130 to 150 µm, or 140 µm, followed by two consecutive segments having a longer length, *e.g.* the double of the shorter length, such as *e.g.* 260 to 300 µm, 270 to 290 µm, or 280 µm. Thus, a structure as depicted in Figure 12d can be formed.

Means and methods for producing a microfluidic chamber as described above are not particularly limited and are known in the art. In particular, the surface opposing the solid support can be made of polydimethylsiloxane (PDMS) by molding in a prefabricated photolithographically patterned mold. The finished product can then be bonded to the solid support. In a specific and preferred example, the microfluidic chamber is produced as described in Examples, Experimental procedures.

In preferred embodiments, the method of the present invention further comprises, after step (c), the step of identifying solid support-bound cells or virus particles of interest microscopically. Suitable microscopy techniques are not particularly limited and are known in the art. They include, for example conventional light microscopy and/or fluorescence microscopy techniques.

In a further preferred embodiment, the method of the present invention further comprises, after step (c) and after microscopic identification as described above, the step of isolating one or more individual immobilized cells or virus particles from the chip. This can be achieved by means known in the art, *e.g*. by aspiration of individual cells with the help of a capillary micropipette, preferably a micropipette that is mounted on a micromanipulator. In the context of this embodiment, the surface opposing the solid support, *e.g.* as provided in the microfluidic chamber described above, forms a removable lid to allow for access to the immobilized cells or virus particles.

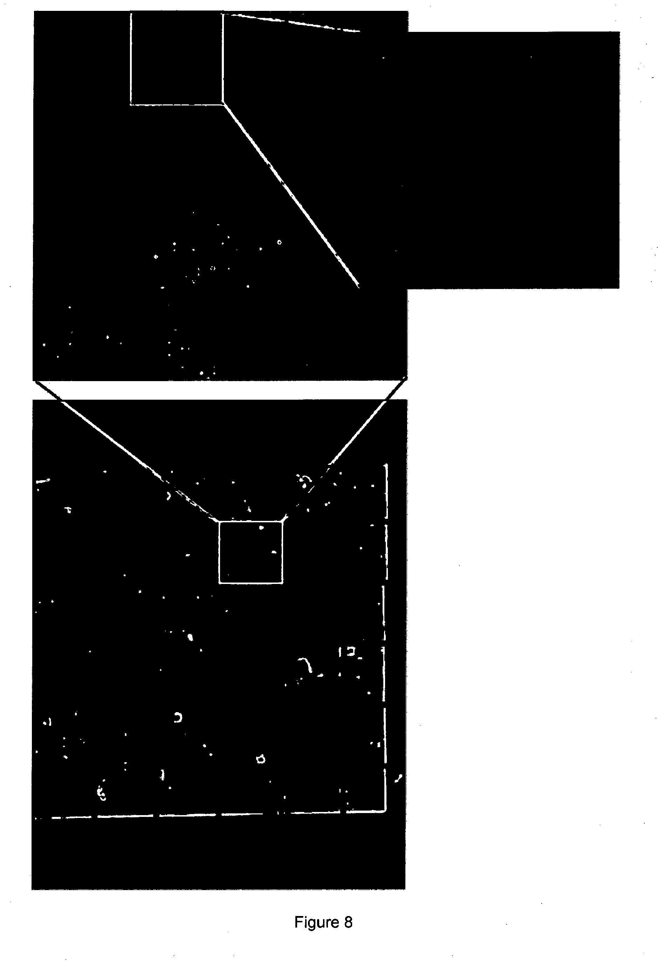

In preferred embodiments, the above additional methods steps of identifying and isolating solid support-bound cells or virus particles is performed in an automated manner. Respective means for automatic identification and isolation are not particularly limited and are known in the art. They include for example the CellaVista system (Roche, Switzerland) or the CellSelector system (ALS Germany) (Fig. 8).

In a preferred embodiment, step (c) of the method of the present invention is performed at elevated temperatures, *i.e*., temperatures higher than room temperature, e.g. at 28 to 40°C, preferably at 32 to 38°C, more preferably at 36 to 38°C, e.g. at 37°C, in order to promote binding of cells or virus particles of interest to the second binding agent. In particular, it has been found that binding of cells or virus particles is unexpectedly improved at elevated temperatures. This can be realized *e.g*. by mounting the solid support to a heating device such as a heating plate. Alternatively, a heating device could be integrated directly into the solid support.

In preferred embodiments, the method of the present invention is performed using microfluidic chambers or devices as described above or as described in further aspects of the present invention hereinafter.

Further, described herein is a microfluidic chamber, comprising:
(i) a solid support comprising an array of defined isolated spots of a solid support-bound binding agent; and
(ii) a surface opposing the solid support, said surface having transverse structures that facilitate chaotic mixing of the fluid containing the cells or virus particles of interest when said fluid flows along said surface, wherein said transverse structures are surface ridges having a herringbone-structure.

In this aspect, all definitions and embodiments described above for the method of the present invention apply in an analogous manner.

In particular, the microfluidic chamber itself, methods for producing the same, the solid support, the array of defined isolated spots of a solid support-bound binding agent, said binding agent, the surface opposing the sold support, the transverse structures that facilitate chaotic mixing of the biological fluid, and the herringbone-structure are preferably as defined above for the method of the present invention.

In specific examples, the transverse structures that facilitate chaotic mixing of the fluid containing the cells or virus particles of interest when said fluid flows along said surface are preferably surface ridges as defined above for the method of the present invention, *i.e*., surface ridges having a herringbone-structure as defined above for the method of the present invention. Further, the surface opposing the solid support preferably forms a removable lid. Furthermore, it is preferred that the areas on the solid support surrounding the defined isolated spots of solid support-bound second binding agent, as well as all additional surfaces of the chamber, are passivated as described above for the method of the present invention. Furthermore, the binding agent, which is preferably streptavidin, is preferably fluorescently labeled.

In a second aspect, the present invention relates to a device for the isolation of cells or virus particles of interest expressing one or more predetermined surface marker(s), comprising:
(a) a microfluidic chamber comprising:
   (i) a solid support comprising an array of defined isolated spots of a solid support-bound binding agent; and
   (ii) a surface opposing the solid support, said surface having transverse structures that facilitate chaotic mixing of the fluid containing the cells or virus particles of interest when said fluid flows along said surface wherein said transverse structures are surface ridges having a herringbone-structure;
   and
(b) a capillary micropipette.

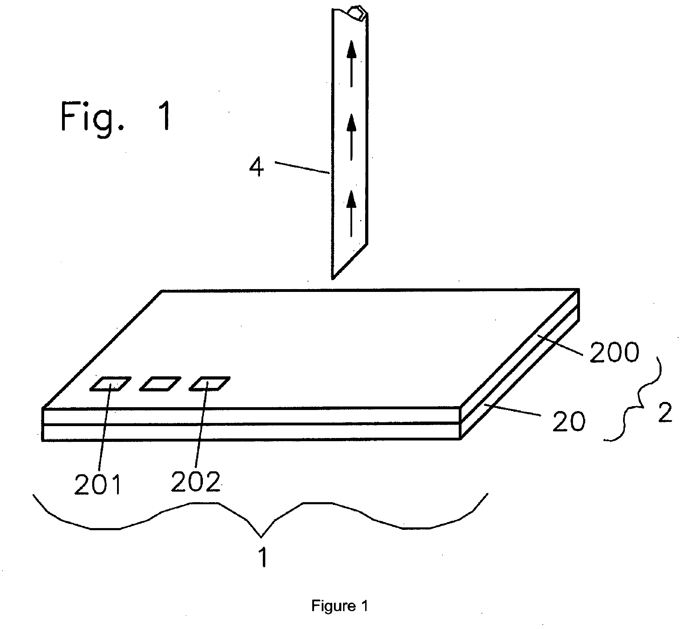

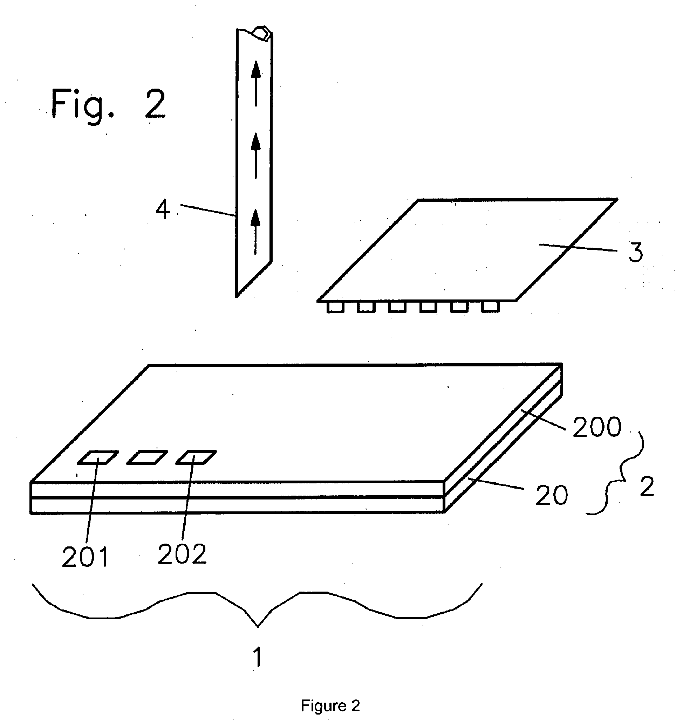

The microfluidic chamber of the device of the present invention is the microfluidic chamber as defined above. A schematic setup of the device of the present invention is shown in Figures 1 and 2.

According to this aspect of the present invention, cells or virus particles are isolated using a capillary micropipette, *i.e*., by aspiration with said capillary micropipette. Respective micropipettes are not particularly limited and are known in the art. They include *e.g.* glass capillary micropipettes having an inner diameter of from 10 to 100 µm, preferably from 20 to 80 µm, from 30 to 50 µm, *e.g*. 40 µm. Preferably, the micropipette is mounted to a micromanipulator as known in the art.

In preferred embodiments, the device of the present invention further comprises means for the automatic identification of solid support-bound cells or virus particles of interest, and/or means for the automatic isolation of immobilized cells or virus particles from the solid support.

In another preferred embodiment, the device of the present invention further comprises means for heating the solid support, *e.g.* a heating plate.

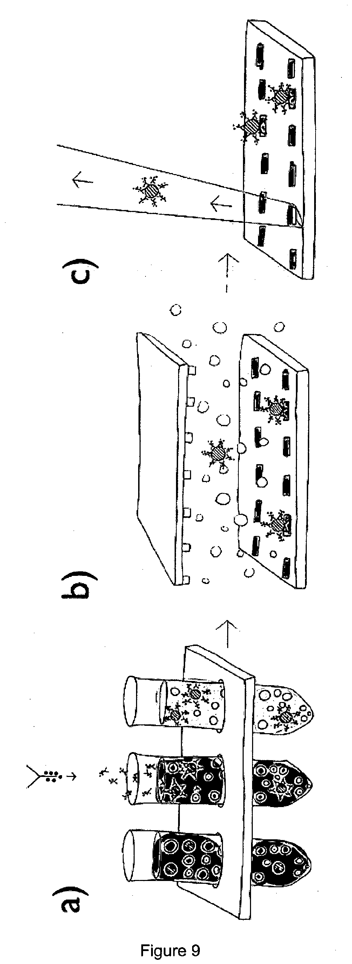

Here, a capture strategy is presented based on micropatterns that offers high specificity, large throughput and easy access to captured cells for single cell analysis (Fig. 9). A streptavidin microarray of e.g. 10×40 mm² functions as capturing platform for cells pre-labeled with *e.g*. biotinylated antibodies. A microfluidic PDMS chip with an integrated herringbone structure introduces transverse components in laminar flow conditions to guarantee contact between labeled cells and micropattern during incubation. To proof the principle, cells of the cell line MCF-7 have been sensitized with biotinylated CTC-specific antibodies (EpCAM) and spiked into blood cells or a suspension of untreated MCF-7 cells. Although using this well-studied system as model, this method is open for any other biotinylated markers and even feasible for antibody cocktails.

One advantage of pre-labeling target cells with an antibody cocktail instead of anchoring one type of antibody onto the surface of the capture device is that the fabricated chip has no limitation concerning the type of cell (or virus particle) that is to be captured at the same time. In fact, any cell (or other target) that can be sensitized by biotinylated antibodies in a prior incubation step can be captured with one chip, allowing for great flexibility in this approach: *E.g*. only the general biotin conjugate will bind to the streptavidin. The binding mechanism is based solely on the interaction between *e.g.* biotin and streptavidin. Even if the incubation protocol varies and different antibodies are used, the fluidic parameters do not change. A further advantage of pre-labeling the target cells is an increased antibody stability and increased interaction of the antibodies with the solid support, thus facilitating cell capture on a plane smooth surface provided by the solid support.

Using a plane smooth surface provided by the solid support according to the present invention for cell capturing provides the additional advantage of an easy isolation of cells after capture for further analysis without excessively disturbing the cells. When using the solid support in combination with an opposing surface having transverse structures that facilitate chaotic mixing, the efficiency of cell capture is comparable to the use of structured capture surfaces lacking this advantage.

Capture approaches based on microfluidic chips aim primarily at cell quantification, however, immunocytochemistry and fluorescence *in situ* hybridization (FISH) can be conducted on captured cells inside these closed systems. Due to the three-dimensional inner architecture that is homogeneously coated with functional molecules captured CTCs cannot be extracted and individually investigated any further. Immobilizing CTCs on a plane surface according to the present invention has some advantages if further investigation needs to be performed. The approach taken in the present invention immobilizes cells exclusively on the functional micropattern; other surfaces like the herringbone-structured ceiling and the channel walls are passivated with *e.g*. BSA preventing cells to bind there. Having the captured CTCs on a flat surface allows picking single cells *e.g*. by a micromanipulator and capillary micropipette.

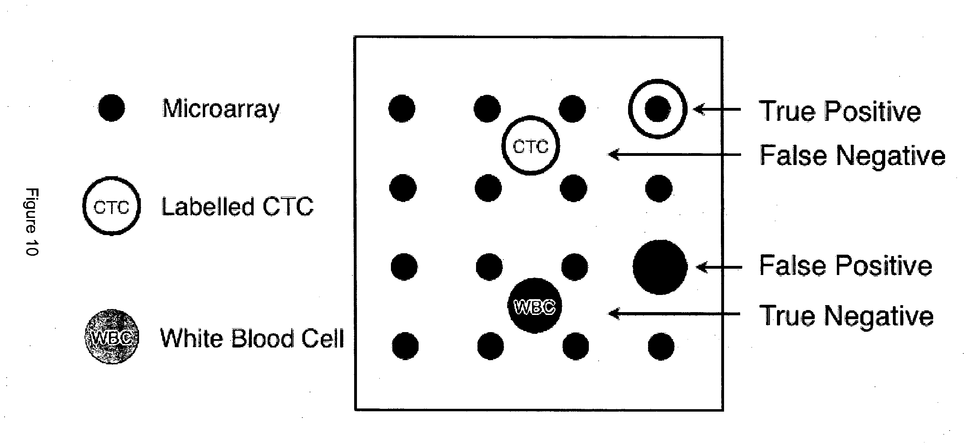

In contrast to other cell capture chips that have a homogeneous antibody coating on the inner walls, the capturing strategy according to the present invention is based on spot microarrays, offering intrinsic specificity enhancement by co-localization detection (Fig. 10). Although cell capture chips are flushed with buffer after the binding procedure, some negative cells can and will always remain. Since *e.g.* non-CTC cells outnumber actual CTCs by several orders of magnitude, false positive (*i.e.* non-specific adhering "captured" cells) are expected to be a serious issue (cf. Fig. 10). However, the co-localization of the approach taken in the present invention leads to a direct sortation into positive and negative cells. After sample incubation, cells that are located on *e.g.* fluorescently labeled streptavidin spots are expected to carry the biotin anchor and are therefore positive for EpCAM or other corresponding biotinylated antibodies, respectively. In contrast, true negative events, *i.e*. cells that are located in between streptavidin dots, can be discarded for further analysis, as their chance to be false negative is low (cf. Table 1 for experimental data). Thus, just knowing the position of the dots can verify the capture of a specific cell of interest. Further fluorescent staining is not needed, however, can be performed within the microfluidic chip if desired. In the present invention, both antibody positive and negative cells remaining on the microarray were stained to clearly evaluate values for efficiency and specificity. Moreover, capturing cells of interest on a defined spot microarray further facilitates the picking of cells for further analysis, e.g. by the advantage of having defined picking positions aligned with the grid of spots.

The genomic analysis and quantification of circulating tumor cells in the blood of cancer patients will play an important role for a precise tumor diagnosis and monitoring during and after treatment. Introduced as liquid biopsy of cancer, the analysis of CTCs as metastatic cells offers additional information of the primary tumor that are not accessible by other means. Furthermore, the patient is aggravated from first surgical interventions. During cancer treatment, *e.g.* chemo- or radiotherapy, physicians are able to evaluate its impact by the quantification of CTCs.

The strategy in cell capture according to the present invention immobilizes and aligns cells on a microarray for intrinsic detection validation. Streptavidin spots of 10 µm in diameter patterned over an area 10×40 mm² function as capturing platform for cells pre-labeled with biotinylated antibodies. To maximize the chance of contact between cells and the microarray, the microarray is encapsulated within a microfluidic PDMS chip carrying a herringbone structure in its channel top wall that causes a chaotic stirring flow. In contrast to other cell capture chips, this approach allows identification of cells without secondary labeling due to a high specificity and intrinsic additional check against unspecific adhesion by the co-localization with the fluorescent capture spots and provides the ability to pick single cells with a micromanipulator to perform further single cell analysis. Furthermore, the preincubation allows for free choice of used antibody in our approach, without need for adjustments. For example, besides EpCAM, other biotinylated or otherwise tagged CTC-specific surface proteins can be targeted. This idea will lead to the application of an antibody cocktail to include EpCAM negative CTCs for capture and could even be expanded to any other antibody target desired to be captured. This proof-of-principle study shows the value of this approach for the ongoing development of microfluidic cell capture chips.

The figures show:
Figure 1:
   Cell capturing device according to the present invention
   A cell capturing device (1) is shown. The cell capturing device (1) comprises a cell adhesion device (2) containing a substrate (20). The substrate is coated with a protein (200). Within the protein coating there are dots of a substrate-bound binding agent (201, 202, ...) that are bound to the protein (200). Also the cell capturing device (1) bears a micromanipulator (4).
Figure 2:
   Cell capturing device according to the present invention
   Another embodiment of the cell capturing device (1) is shown. The cell capturing device (1) comprises a cell adhesion device (2) containing a substrate (20). The substrate is coated with a protein (200). Within the protein coating there are dots of a substrate-bound binding agent (201, 202, ...) that are bound to the protein (200). Also the cell capturing device (1) bears a micromanipulator (4). Additionally the cell capturing device (1) bears a removable structured lid (3) which can be used to close the cell adhesion device (2).
Figure 3:
   Principle of using the cell capturing device (1) as well as a method to capture cells.
   First in step a) a sample (5) containing cells (51) and cell (52) is provided. The cell containing sample (5) is in step b) incubated with a labeled antibody (6) yielding to a labeled cell-antibody construct (7) containing sample (8). In step c) the labeled cell-antibody construct (7) containing sample (8) is exposed to the cell adhesion device (2) whereby a labeled CTC-antibody-dot construct (9) is formed by reaction of the labeled cell-antibody construct (7) with the dots of a substrate-bound binding agent (201, 202, ...). In Step d) the cell-antibody-dot construct (9) is removed from the substrate (20) with the micro-tip (4) leaving an empty spot on the substrate (210). The labeled cell-antibody-dot construct (9) is then collected and/or analyzed.
Figure 4:
   Examples of labeled (green/blue; *e.g*. arrow 1) and untreated (only blue; *e.g*. arrow 2) cancer cells on microarravs using a microfluidic setup according to the present invention.
   All cells were mixed in 1 mL PBS and pumped through the HB-chip. The scale bars equal 10 µm.
Figure 5:
   Analysis of feature size.
   Fluorescence micrograph of the FITC channel showing a biotin-4-fluorescein array after printing (upper panel). The size distribution of the feature analysis performed with imageJ is given in the histogram in the lower panel. The average dot area is 77 µm² ± 11 % or (8.8 ± 1.0) µm in edge length.
Figure 6:
   Feature sizes at five different array positions.
   These fluorescence micrographs were taken of one PPL generated microarray of 1.5 x 1.5 cm² area in each corner and in the array center. The pen's spacing is 50 µm. Besides the micrograph in the bottom left of the sample, the average feature size is in between 58 µm² and 64 µm²; indicating that PPL is able to pattern large homogeneous arrays.
Figure 7:
   Four different experiments with either whole blood or only leukocytes incubated on a coverslip carrying the microarray, and MCF-7 cells either labeled before spiking into the blood cell suspension or labeled in the blood cell suspension. The corresponding path to the micrographs is indicated in the diagram. All alternatives show a clear affinity of the labeled cancer cells (2° AB staining to Anti-EpCAM) to the dots. The circle indicates the most desirable way: Cancer cells that are labeled in whole blood and incubated on the microarray with whole blood can be co-localized on the pattern.
Figure 8:
   Automated microarray analysis.
   Cell identification can be performed automatically by using systems like CellaVista (Roche, Switzerland). The chip (here: a cover slip) is placed into the microscope and scanned. As the cells are co-localized on one plane, the cells remain in focus - even over centimeters. This image combines 576 x 2 micrographs of the Texas Red and DAPI channel in one image. Other systems like the CellSelector (ALS Germany) are able to automatically decide whether a cell is located on a red dot, save the x/y-coordinates and automatically pick the cell from the pattern. One square of 4 x 4 sub-images has a side length of about 2.5mm, 1 square of the subimages has a side length of about 600µm, and the last zoom-in stage shows about 500µm of sidelength.
Figure 9:
   Cell capture and extraction.
   The present invention is based on the labeling of e.g. circulating tumor cells in a blood sample with a specific biotinylated surface antibody, e.g. the epithelial cell adhesion molecule (EpCAM) with a biotin conjugate. Red blood cells and antibody excess is eliminated by following a density gradient protocol in a second step (a). By pumping the cell suspension through a microfluidic chamber that has a herringbone structure in its channel top wall to generate a stirring flow and a streptavidin micropattern on the bottom surface, biotin-labeled cells get immobilized on the pattern due to the biotin-streptavidin interaction (b). Single cell analysis, *e.g.* whole genome amplification, can be performed after the cells are quantified, optically localized and extracted from the pattern with a micro-capillary (c). Sample observation can be carried out with totally automated systems.
Figure 10:
   Definition of efficiency and specificity.
   Antibody positive and negative cells will be in the patterned area after a capture experiment. This illustration describes the four possible cases: true positive events (cell is on a dot), false negative events (a cell is in between dots), false positive events (a negative cell is on a dot) and true negative events (a negative cell is in between dots). The efficiency is defined as the ratio of true positive events over all cells on the array. The specificity is the ratio of true negative cells over all negative cells on the array.
Figure 11:
   Setup of a cell capturing experiment with a microfluidic device according to the present invention.
   A syringe microfluidic pump is positioned vertically to prevent air bubbles to enter the system and to enforce the cells to directly enter the syringe's exit. A threeway cock simplifies the substitution of syringes. A d=1/32" tube connects the syringe with the inlet of the microfluidic chip that is placed on a heatable plate. The outlet tube leads into an Eppendorf tube to collect the cells that transmitted the chip.
Figure 12:
   Capturing and immobilization of biotin-labeled circulating tumor cells (CTC) by microarrays.
   (a) Array fabrication is processed by polymer pen lithography (PPL). The polydimethylsiloxane (PDMS) tips are coated with biotin-4-fluorescein and then precisely navigated to the surface to generate the micropattern on a 4 cm² area. (b) This schematic describes the overall generation of the streptavidin array. A microscopy glass slide functionalized with a layer of bovine serum albumin (BSA) allows covalent binding of the fluorescein-tag by photobleaching. A topographically flat biotin-array is achieved after washing with PBS. After mounting the fluidic system, the microarray is further functionalized with streptavidin/cy3. (c) A photograph of the microfluidic system that is fixed on a hot plate. The microarray on the microscopy slide is marked with black dots that correspond to the fluidic chamber, too. (d) The brightfield image shows the top channel wall of the microfluidic chip in (c) that is responsible for a chaotic stirring stream. The scale bar equals 200 µm. (e) The fluorescence micrograph shows a labeled cancer cell (DAPI staining, alexa488 2° staining for anti-EpCAM) trapped in the fluidic system on a streptavidin/cy3-dot. The micrograph visualizes the herringbone structure in the cap as a shadow. The scale bar equals 50 µm.
Figure 13:
   Top view of a PPL stamp during lithography.
   PDMS pens formed as pyramids are wetted with biotin-4-fluorescein and brought in contact with a BSA coated microscopy slide to generate the microarray. The squares in the middle of each pen indicate the surface contact area. Scale bar equals 40 µm. The pitch of pyramidal tips is 80 µm.
Figure 14:
   Topographically flat streptavidin features.
   The fluorescence micrograph shows a streptavidin¥cy3 array in liquid (upper panel). The brightfield micrograph of the same position with loaded cells (lower panel). It is not possible to identify the streptavidin pattern. Scale bar equals 20 µm.
Figure 15:
   Examples of captured cancer cells (all labeled) on microarrays.
   10⁶ cells of the cell line MCF-7 were sensitized with 1 µg anti-EpCAM for 40 min. 200 µL suspension was then incubated on a cover slip carrying the streptavidin microarray. The temperature was kept at 37 °C. Scale bar equals 40 µm.
Figure 16:
   (a) The micrographs show overlays of the brightfield, texas red, FITC and DAPI channel illustrating the streptavidin-pattern in red (*e.g.* arrow 1), the cell nuclei in blue (*e.g*. arrow 2) and anti-EpCAM in green (*e.g*. arrow 3). Two cells of the cell line MCF-7 are captured. A glass micropipette with a diameter of 40 µm is positioned above the cells before picking them from the pattern. Further gene sequencing of the cells can be carried out after processing whole genome amplification (WGA). The inlet (b) shows residues of the outer cell membrane after picking. Scale bar in (a) equals 30 µm, in (b) 15 µm.
Figure 17:
   Capturing of sensitized cells by microarrays.
   (a) MCF-7 cancer cells labeled with biotinylated anti-EpCAM (green; *e.g*. arrow 1) are mixed with leukocytes (DAPI staining blue; *e.g.* arrow 2) and incubated on the streptavidin pattern (red, *e.g.* arrow 3). Whereas the positive cells show a clear attraction to the dots, the biotin-negative leukocytes do not show a reaction to the pattern. The dot spacing of 20 µm is small enough to allow cells to bridge dots. By increasing the dot spacing like in (b), the positive cells can be individually captured and positioned on one dot. (c) A homogeneous antibody coating does not allow a conclusion about the binding cells only by the position. The micrograph shows biotin-negative cells (DAPI staining only) and biotin-positive cells (secondary staining in green) randomly binding on the surface. Cell identification cannot be carried by the position. Scale bars equal 20 µm in all images.
Figure 18:
   Performance of the presented microarrav chip.
   The upper panel diagram faces recovery, efficiency and specificity of three different spiking / dilution experiments. The recovery describes the total performance of the respective experiment. It becomes clear that all three experiments have a similar behavior showing recoveries of 40 to 60 %, an efficiency of 60 and 80 % and a specificity of more than 85 %. The temperature has a major influence on the chip's performance as the cells lose viability in cold conditions. The average efficiencies of different experiments are plotted against the chip's temperature. The efficiency decreases from 70 % to 40 % when lowering the chip's temperature from 37 °C to 18 °C (middle panel). The lower panel compares the amount of CTCs found in patients' blood samples by the CellSearch setup compared to the chip of the present invention. In patients 1 to 3 both approaches detect cells (the fact that more cells are detected in CellSearch is due to the presence of a lot of apoptotic CTCs in the samples that will be sorted out already during enrichment in the present invention), however even CTCs (confirmed by subsequent staining to be true positives) in samples that CellSearch deemed negative could be detected.

The present invention will be further illustrated in the following examples without being limited thereto.

### Examples

### Experimental procedures:

### Micropattern Fabrication

Biotin microarrays were generated by Polymer Pen Lithography. Briefly, the polydimethylsiloxane (ABCR, Karlsruhe, Germany) stamp is molded from a silicon master that was initially generated by photolithography. Oxygen plasma treatment renders the stamp's surface hydrophilic which is needed to homogeneously coat the pyramided tips when approaching the stamp into a stamp pad filled with ink. The stamp pad is a piece of silicon covered with biotin-4-fluorescein ink (Sigma Aldrich, Germany). The stamp is attached to the holder of an NLP2000 system (Nanolnk, Skokie, IL, US) that offers a stage able to perform movements over some centimeters with sub-micrometer precision. Depending on the pen's spacing, either several approaches are carried out to realize the pattern with the desired parameters. The stamp itself has an area of 1x1 cm². Pen inking is carried out at 70 % relative humidity (RH), lithography at 50 % RH.

A microscopy slide (Menzel Gläser, Germany) acts as substrate for the microarray. Prior cleaning is performed with sonication in chloroform, isopropanol (Merck, Germany) and deionized water (18.2 MΩcm) for 10 min each. The slides were then immersed in a solution of bovine serum albumin (BSA, Sigma Aldrich, Germany) and phosphate buffered saline (PBS, Sigma Aldrich, Germany) of a concentration of 3 % w/V. After 45 min, the slides were dipped in PBS 10 times to remove the excess of BSA from the surface and dried with nitrogen. Inking of the PPL stamps was carried out with the stamp pad method. Cleaned pieces of a silicon wafer were coated with the respective ink mixture by depositing 10-15 µL of ink onto the bare surface by a pipette and allowing the droplet to spread. Then the polymer pens were approached onto this silicon wafer (either by making 10×10 dot patterns with dwell time 1s or by making arbitrary lines) for inking at RH 70 %. Immobilization of the biotin-4-fluorescein micropattern is carried out with a UV lamp at 350 nm for 45 min. The slides are again dipped into PBS 10 times to remove excess and dried with nitrogen.

### Fabrication of the Microfluidic Device

Device fabrication was accomplished by a rapid, low-cost micromolding technique. A 4" glass wafer was used as substrate material for the mold. The inverse of the microfluidic structures were formed within a biocompatible dry film resist (Ordyl SY330, Elga Europe, Italy) with a thickness of 55 µm. For the mold fabrication, the first resist layer was laminated onto the glass substrate at a temperature of 95 °C, and photolithographically patterned. To achieve the desired herringbone structure in the microfluidic channel top wall, a second layer of dry film resist was laminated onto the structure and patterned, resulting in a total height of 110 µm. After resist development, liquid, degassed PDMS was poured over the finished mold. Once solidified, PDMS was peeled off and holes for fluid connections were punched. In a next step, an oxygen plasma treatment of the bottom side of the PDMS was applied. The PDMS was bonded to the micropatterned microscope glass slide. Finally, tubings with syringe needles were fitted into the punched fluid connector holes to finish the microfluidic chip.

### Microfluidics and Chip Functionalization

The microfluidic chip is connected with 1/32 inch tubes that are inserted into provided inlet and outlet ports. The ingoing tube is connected to a 3-way-cock that is again connected to a syringe. A microfluidic syringe pump (NE-1002X, FisherScientific) is used to reproducibly pump the liquids through the chip. To allow a smooth wetting of the whole chamber and to block unspecific protein binding at the channel walls, 100 µL of bovine serum albumin (1 % w/V) and Tween20 (1 % V/V) are pumped into the chip and incubated for 30 min. Then, 200 µL streptavidin/cy3 in PBS (0.5 % V/V) replaces the prior solution to bind streptavidin to the biotin pattern. The chip is ready-to-use after flushing with 500 µL PBS.

### Blood Sampling

4 mL whole blood from a healthy donor was stored in an EDTA tube. A general density gradient protocol was applied to separate red blood cells and mononuclear cells: Blood was given in a falcon tube and filled with 30 mL HBSS (Biochrom) and centrifuged at 400g and 4 °C for 10 min. The cell pellet was resuspended in 30 mL PBS and 20 mL Ficoll (GE Healthcare) were added. The mixture was centrifuged at 400g at 4 °C for 30 minutes. The interface and supernatant, containing the mononuclear cells (*i.e.* leukocytes), were transferred to a new 50 ml falcon tube. The tube was filled with PBS and centrifuged at 400g at 4 °C for 10 min. Supernatant was discarded and cell pellet was resuspended in 1 mL 1×H-Lysis buffer (R&D Systems) and incubated for 3 minutes with gentle shaking at room temperature. 30 mL PBS was added and sample was centrifuged again at 400g for 10 minutes at 4 °C. Supernatant was discarded and pellet was resuspended in warm PBS to spike the biotinylated MCF-7 cells into the suspension.

### Cancer Cell Sensitization

Cells from the cancer cell line MFC-7 (SKBR-3) were cultured in Dulbecco's modified eagle medium (DMEM) including 10 % (5 %) fetal calf serum (FCS) at 10 % (5 %) CO₂ in a 25 cm² flask. Cells were washed with warm PBS and detached with trypsin after 2 min incubation and rinsed with warm DMEM. After centrifugation, the cells were re-suspended in warm BSA/PBS 0.1 % w/V and counted. 100.000 cells in warm PBS/BSA were then incubated with either 0.5 µg biotinylated Anti-EpCAM (VU-1D9, Abcam, UK) or biotinylated Anti-HER-2 (3B5, Abcam, UK) in a shaker (Eppendorf Thermomixer Comfort, Hamburg, Germany) revolving at 300 rpm at 37 °C for 40 min. The cells are then centrifuged and suspended in warm PBS. The ratio of dead cells is counted before spiking. To achieve a precise number of spiked cancer cells, the suspension is diluted to 10.000 cells/mL; 5 drops of 10 µL suspension are then pipetted on a glass slide and the cells are counted.

### Capturing Experiments

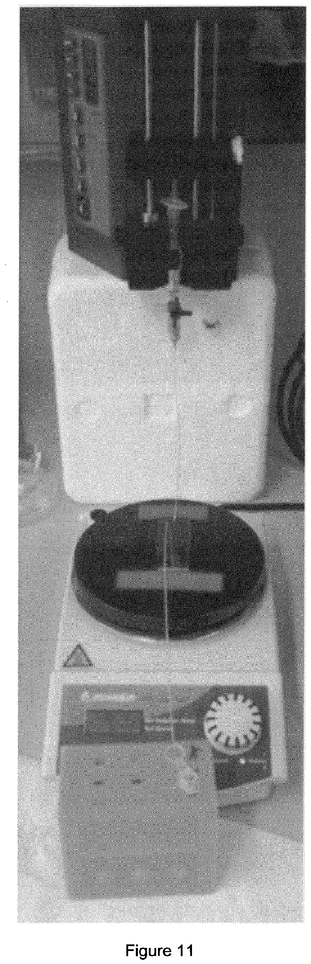

Positive and negative cells are mixed in 1 mL PBS and pipetted into a 3 mL syringe (BD Bioscience) that is covered with aluminum foil to hold a temperature of 37 °C. To prevent air bubbles to enter the system and to allow the cells to enter the syringe's exit directly, the microfluidic pump is placed vertically (Fig. 11). The pump is set with individual parameters according to the given protocol. A flow rate of 20 µL/min is most commonly used. The chip is placed on a hot plate (Heidolph MR Hei-Tec, Schwabach, Germany) set to 37 °C. The cells leaving the device at the exit are collected in an Eppendorf tube. Cells not adhering in the microarray are flushed with 500 µL warm PBS (50 µL/min) and collected in the same eppendorf tube. These cells are then cytospinned on a superfrost glass slide (Menzel-Gläser, Germany) at 2000 rpm for 3 min, fixed with 4 % paraformaldehyde (PFA), stained for the initial EpCAM-label plus DAPI and quantified with a fluorescence microscope. At least five micrographs of low magnification (5×, 10×) of the cytospinned area are taken and average numbers of cells in the whole area are calculated. These numbers are used to obtain the overall recovery. The cells in the chamber can be fixed with PFA for 15 minutes and be stained with DAPI (1:1000) and a secondary antibody against mouse carrying an Alexa488-fluorophore (1:200). To get rid of the excess of staining antibodies, the chip is flushed with another load of PBS (500 µL, 50 µL/min). The number of bound cells is then obtained by counting with an inverted fluorescence microscope. For extracting bound cells with a capillary attached to a micromanipulator, the cells are not fixed. The microfluidic PDMS chip can be cut open with a scalpel to get access to the cells.

### Example 1:

### Design of a microfluidic chip and an integrated microarrav

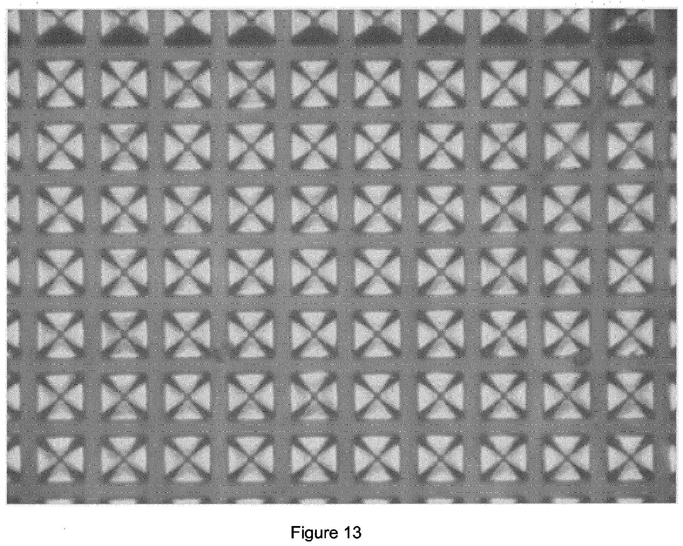

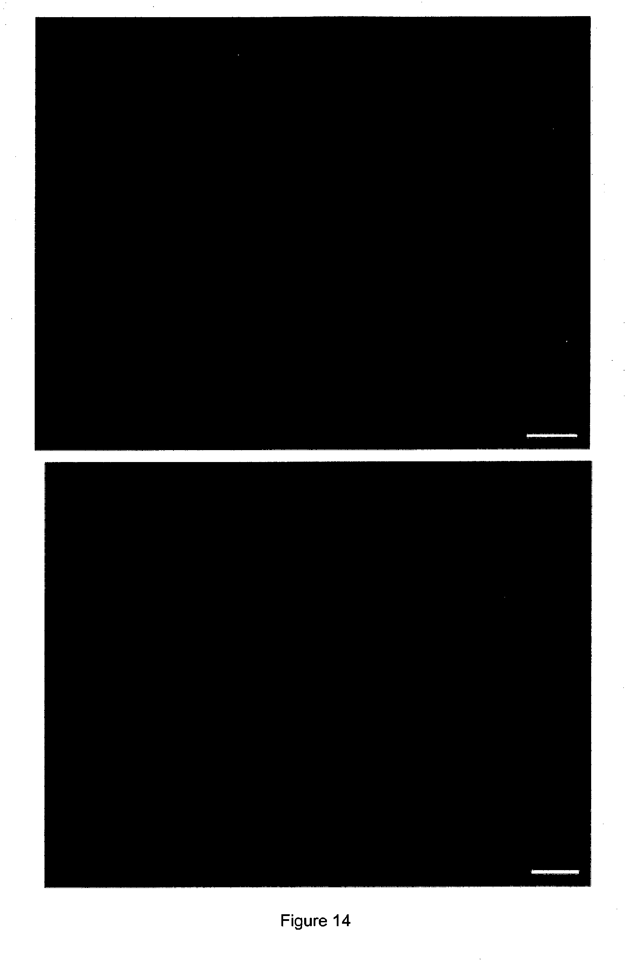

The capturing strategy presented here is based on a functional dot pattern combined with a microfluidic chaotic mixing system. A standard microscopy slide is coated with bovine serum albumin (BSA) to create both a passive and integrative interface: passive, as BSA prevents proteins and cells from unspecific binding; integrative, as fluorescein can bind covalently to BSA molecules induced by photobleaching. The latter is exploited to generate a chemisorbed dot pattern on the microscopy slide that is stable under streaming liquid condition. Lithography is carried out by polymer pen lithography (PPL), a high throughput technique with full pattern flexibility. Figure 12a presents a schematic of the PPL process: a two dimensional array of polydimethylsiloxane (PDMS) pyramids is brought in contact with a piece of silicon coated with biotin-4-fluorescein acting as an ink pad. Capillary forces cause a wetting of the pens that are then navigated above the BSA coated glass slide. The dot pattern is generated by automatically approaching the stamp (Fig. 13) onto the surface. The piezo-assisted navigation of the stamp or stage respectively guaranties full pattern flexibility by bringing the stamp into contact with the surface more than once to induce several dots subsequently with each pyramidal tip and allows a homogeneous surface covering of 1×4 cm² within two minutes, *i.e.* 640.000 features in case of a 25 µm dot spacing. Figure 12b illustrates the following steps to achieve the streptavidin pattern. Bleaching the fluorescein-fluorophore leads to a covalent photochemical binding to the BSA and leaves a topographically flat biotin array after washing (Fig. 14).

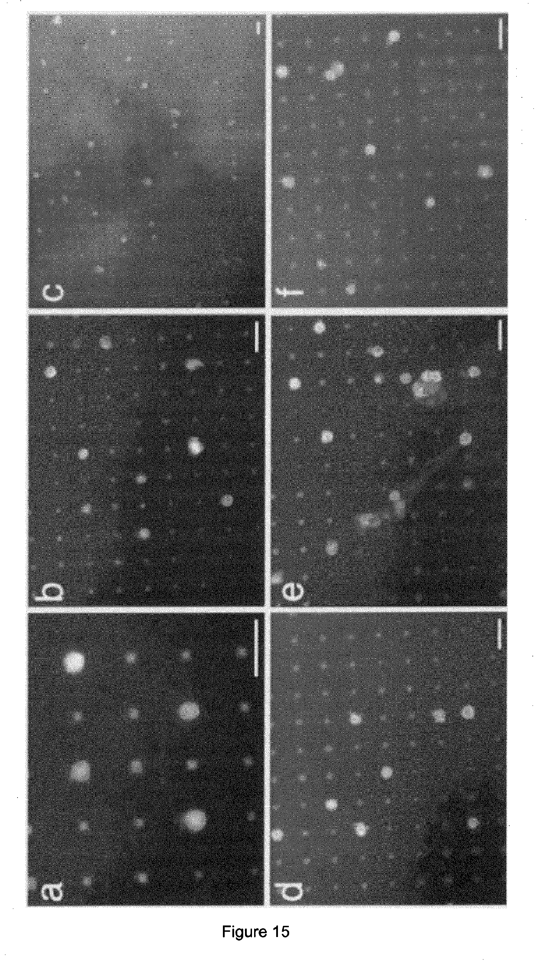

The microfluidic PDMS chip is casted from a prefabricated mold and covalently bonded to the microscopy slide carrying the microarray after oxygen plasma treatment. The array matches the microfluidic channel design with its herringbone topography in the top wall. The microdevice can be stored for months before application since the biotin pattern is stable in air and not prone to degradation as compared to antibody patterns. By connecting the microfluidic chip to a syringe, the chamber is flushed with a solution of streptavidin/cy3, which binds to the biotin-array and turns it into the active capture array for biotin-labeled targets. After flushing with PBS to remove excess streptavidin, the device is ready for CTC capture. A photo of the microfluidic device is given in Figure 12c. In order to increase the cell-surface interaction, which is limited in laminar flow conditions, this device has a herringbone shaped ceiling (Fig. 12d). Besides causing a stirring effect in the cell suspension flowing through the chip and hence increasing the probability of cell-surface contact, the structure also distributes the cells homogeneously over the whole pattern, respectively (Fig. 15). The overlaid fluorescence micrographs in Figure 12e shows the streptavidin/cy3 pattern in red and one captured cell from the breast cancer cell line MCF-7 that has been sensitized with biotinylated EpCAM and stained with an alexa488-tagged secondary AB against anti-EpCAM and DAPI in blue. The herringbone structure appears as shadow in the green channel.

### Example 2:

### Identification and single cell extraction

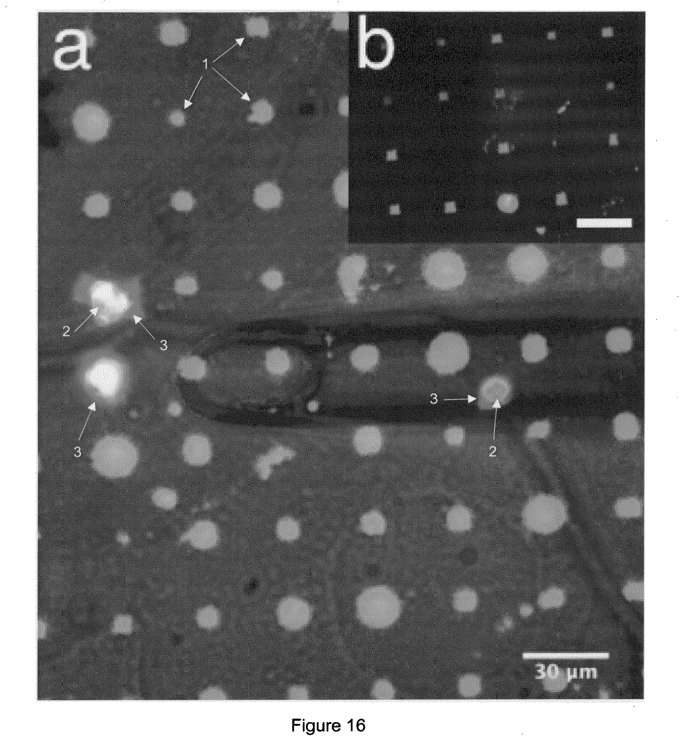

Capture approaches based on microfluidic chips aim primarily at CTC quantification, however, immunocytochemistry and fluorescence *in situ* hybridization (FISH) can be conducted on captured cells inside these closed systems. Due to the three-dimensional inner architecture that is homogeneously coated with functional molecules captured CTCs cannot be extracted and individually investigated any further. Immobilizing CTCs on a plane surface has some advantages if further investigation needs to be performed. The approach taken in the present invention immobilizes cells exclusively on the functional micropattern; other surfaces like the herringbone-structured ceiling and the channel walls are passivated with BSA preventing cells to bind there. Having the captured CTCs on a flat surface allows picking single cells by a micromanipulator. Figure 16a shows an overlay of fluorescence and brightfield micrographs that illustrates the picking procedure. Two trapped cells bind to streptavidin dots; a glass capillary with a radius of 40 µm approaches one cell from the top. The cell is then detached from the dot by putting the aperture over the cell and carefully scratching underneath it. An adjustable inflowing stream loosens the cell and carries it into the capillary. The capillary's tip can then be navigated into a container and an outgoing stream flushes the cell into it. Figure 16b shows the same spot as Figure 16a only after picking the cells from the pattern. The overlay of the texas red, FITC and DAPI channel proves that the two cells have been detached from the dots due to the absence of DAPI. The dots on that the cells bound previously have not been damaged. Little green spots around the streptavidin dots remain though. However, it could be shown that these cells were still intact after picking. As the affinity between streptavidin and biotin is stronger than EpCAM / anti-EpCAM or the force needed to extract EpCAM from the cell membrane, we conclude that these residues belong to anti-EpCAM with or without attached EpCAM proteins.

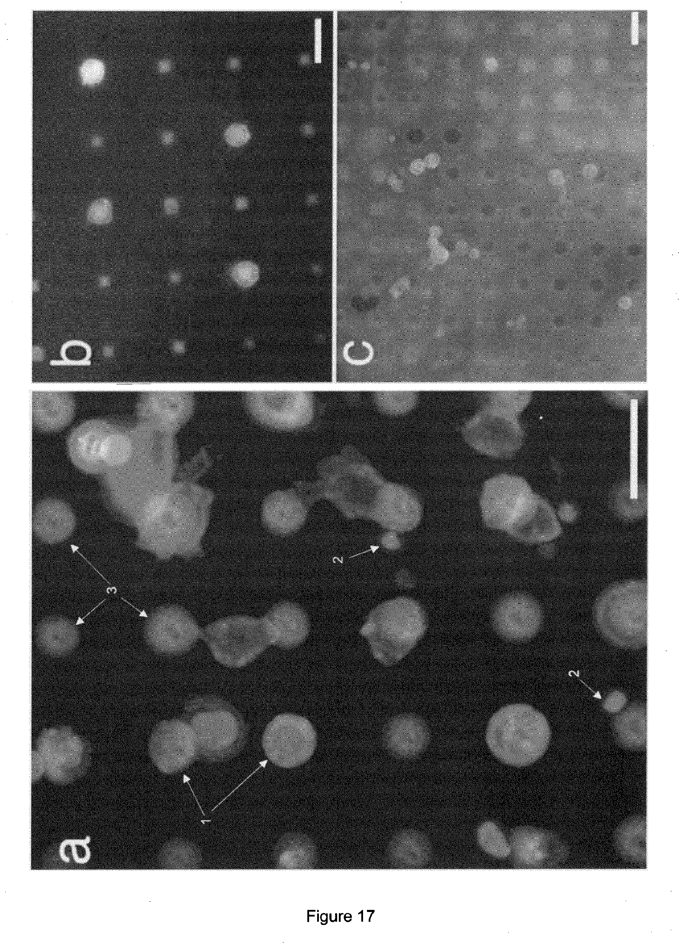

Figure 17a shows labeled cancer cells (MCF-7, secondary staining in green) and leukocytes (nucleus stained with DAPI blue) on a streptavidin/cy3 dot pattern in red. The dot's spacing is 20 µm. The attraction of the pattern to the labeled green cells is clearly visible. Leukocytes that also stand out by their smaller size do not sit on dots. These cells will be neglected for further quantification and analysis. The narrow patterning allows for some cells bridging between two dots. This is a drawback when observing only the brightfield micrograph as these cells would be considered negative. To avoid this, the dot pattern is fabricated with a larger pitch (Fig. 17b). Then, cells can be clearly allocated to dots and considered as positive. Furthermore, the larger spacing between the dots increase specificity as the probability of false positive events can be reduced.

The advantage of facilitating dot patterns as capturing platform becomes clear when referring to Figure 17c. Here, 50 % of MCF-7 cells have been labeled with biotinylated EpCAM; the other half has not been labeled to function as negative cells. When pumping and incubating the cells through a homogeneously coated streptavidin chip, both positive and negative cells remain; even after washing. The unspecifically bound negative cells do not allow any conclusion about the cell type, unless further fluorescent staining is performed.

### Example 3:

### Performance

The low quantity of CTCs in blood is a challenge for all capturing strategies. A relatively large blood volume of several mL needs to be processed to isolate usually some hundred CTCs. When facilitating specific surface proteins as CTC marker, all blood cells need to be brought in contact with an interacting surface. The first experiments to proof the stability and binding dynamics of the micropatterns have been performed with 100 µL cell suspension on cover slips. EpCAM micropatterns have been created by direct protein deposition and it was tried to incubate biotinylated EpCAM on a streptavidin array. However, both approaches did not show sufficient binding results during a short-term incubation of 15 minutes. By sensitizing targeting cells with biotinylated EpCAM one can take advantage of the strong affinity between streptavidin and biotin. Live-imaging experiments show that 80 % of biotinylated cells are trapped on a streptavidin dot immediately after touching. However, using cover slips alone will not solve the volume problem, as most of the blood cells will not have a chance to touch the functionalized surface. By designing a microfluidic chip with a herringbone structure enveloping the micropattern the cell-surface interaction and therefore the binding probability is enhanced.

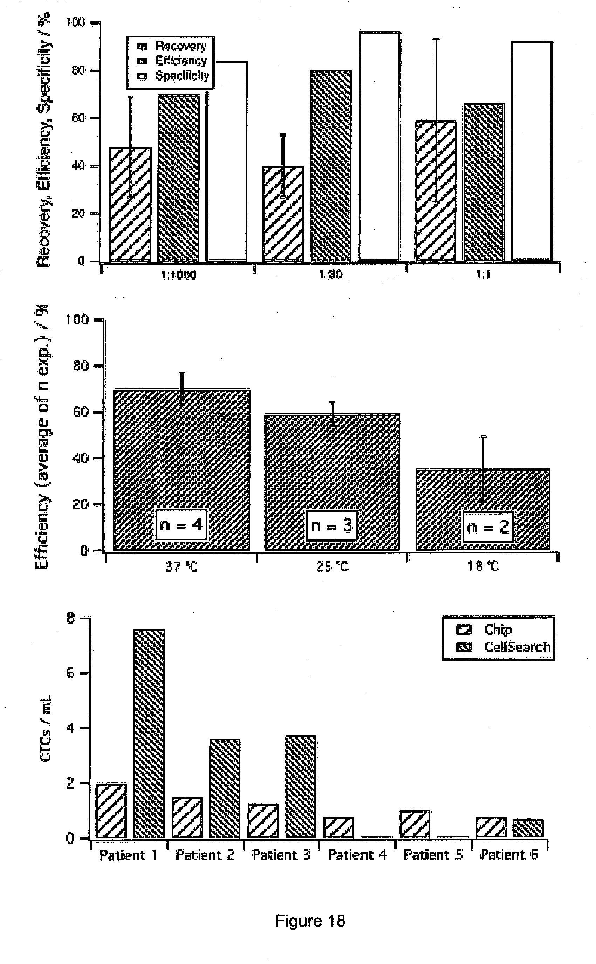

Table 1 presents a set of experiments from a statistical point of view. MCF-7 cells have been sensitized with biotinylated EpCAM and spiked into lysed blood carrying 2×10⁶ leukocytes (#1) or into an untreated cell suspension of MCF-7 cells (#2-4). These suspensions were then pumped through the microfluidic chip keeping the temperature at 37 °C. Binding effects increase when the cells have a longer contact time to a dot, *i.e*. less velocity. Therefore, a herringbone-structured chip was facilitated to mix the cell suspension to allow any cell to get in touch with a streptavidin dot. By interrupting the flow the cell velocity in the chamber decreases which results into a larger reaction time between cells and dots. Following this protocol, we were able to achieve average recovery rates of about 50 % could be achieved. All experiments presented in Table 1 show a high specificity between 86 % and 96 %. The upper panel of Figure 18 visualizes the statistical data and especially points out the homogeneous performance of the chip during different ratios of positive and negative cells.

**Table 1: Performance of four independent capture experiments**

| # | **Recovery** | **Eff.** | **Spec.** | **pos:neg** | # **cells imaged** | **Volume** |
|---|---|---|---|---|---|---|
| 1 | 48 ± 44 % | 70 % | 86 % | 1:1000 | 180 | 1000 *µ*l |
| 2 | 40 ± 32 % | 79 % | 96 % | 1:30 | 83 | 1000 *µ*l |
| 3 | 60 ± 57 % | 66 % | 92 % | 1:1 | 199 | 800 *µ*l |
| 4 | n/a | 64 % | 93 % | 1:5 | 50 | 900 *µ*l |

As can be taken from Table 1, the recovery varies between 40 and 60 %, the specificity is higher than 85 %. All experiments were performed with cells of the cell line MCF-7 that have been labeled with biotinylated anti-EpCAM before. Experiment 1 was carried out with 1 mL lysed blood and 2050 spiked cells. To allow cell binding, the flow of 20 µL/min was interrupted in periods of 2 minutes for 3 minutes each. 99 % of leukocytes passed the chip, whereas 68 % of the positive cells remained in the chamber. The other experiments were performed with non-sensitized cells as negative sample.

The middle panel of Figure 18 shows the temperature dependence of the capture efficiency for different capturing experiments. It was found that the binding efficiency of biotinylated cells incubated on streptavidin patterns on cover slips was low at room temperature. Only when placing the cover slip on a hot plate and therefore maintaining a temperature of 37 °C, a high number of cells bound to the pattern. Running binding experiments with the herringbone-structured microfluidic chip at different temperatures could underline this experience. The binding efficiency decreased about 30 % when using the chip at room temperature instead of 37 °C. Furthermore, the recovery decreases massively as a higher number of positive cells flows through the chip.

### Example 4:

### Additional methods for micropattern fabrication

### Gold/Thiol-Biotin

The gold/thiol substrate/ink system is the most widely employed platform in DPN and PPL. For application in PPL, the PPL stamp is generally plasma cleaned, then spin coated with or completely immersed into the respected thiol solution in ethanol. After drying of the stamp, printing takes place in the same way as described for the fluorescein-biotin patterning. Prior to incubation of the substrate with streptavidin, the substrate is treated with a blocking agent such as BSA.

### Glass/alkyne-group/azide-biotin

### Functionalization of Silica Substrates:

Glass slides were cleaned in piranha acid (3:1 v/v solution of H₂SO₄ and H₂O₂) for 30 min, rinsed with copious amounts of MilliQ water and dried with pressurized air. Caution: piranha acid is a strong oxidizer and a strong acid. It should be handled with extreme care, as it reacts violently with most organic materials. The samples were then immersed in a solution of (3-glycidyloxypropyl)trimethoxysilane (GPTMS) in toluene (1% v/v) for 8 h at room temperature, afterwards rinsed with acetone, and dried with pressurized air. Immediately afterwards, the epoxy ring-opening was performed by immersing the substrates in a solution of propargylamine in acetonitrile (2% v/v) for 8 h at 45 °C. The samples were taken out of the solution, sonicated in ethanol for 5 min, rinsed thoroughly with ethanol and MilliQ water and dried with pressurized air. If the samples were not used directly, they were stored at 4 °C under argon atmosphere.

### Patterning of Alexa Fluor 555 azide and biotin-azide by DPN:

Dip-pen nanolithography was done on a DPN 5000 system (Nanoink, Skokie, USA). A 1D cantilever array containing 26 Si₃N₄ tips was cleaned with oxygen plasma, 10 sccm at 100 mTorr with 30 W for 5 min. The cantilevers were subsequently immersed for 10 min in a solution of Alexa Fluor 555 azide (150 µg mL⁻¹) or biotin azide (PEG4 carboxamide-6-azidohexanyl biotin, 2 mg mL⁻¹), each ink containing 20 mM sodium ascorbate and 10 mM CuSO₄. After inking the cantilever arrays were blow-dried with pressurized nitrogen. Writing of the Alexa Fluor 555 patterns was performed in feedback mode with a velocity of 1 µm s⁻¹ at 74.8% relative humidity and 26.4 °C. The biotin azide dot patterns were generated with a dwell time of 2 s per dot at 74.0% relative humidity and 26.4 °C. After the DPN process for the biotin pattern the sample was blocked with a PBS solution containing 0.5% BSA for 15 min, then washed with PBS three times and incubated for another 15 min with PBS containing 1 vol% Streptavidin-Cy3 (Sigma-Aldrich GmbH, Germany). After that, the sample was washed again three times with PBS, rinsed with MilliQ DI water and blow-dried with nitrogen before inspection with fluorescent microscopy. Prior to incubation of the substrate with streptavidin, the substrate is treated with a blocking agent such as BSA.

In a related method, an azide can be immobilized on the substrate, e.g. via substrate-bound silanes, and a biotin-alkyne bound thereto.

### CVD-Coating with alkyne functionalization and biotin-azide

### CVD polymerization of poly(p-xylylenes):

Poly(4-ethynyl-p-xylylene-co-p-xylylene) was synthesized via CVD (chemical vapor deposition) polymerization in a custom-made CVD polymerization system, and were prepared on substrates including PDMS, gold, PMMA, PS, PTFE, and stainless steel. The synthesis of ethynyl[2.2]paracyclophane followed routes known in the art. Ethynyl[2.2]paracyclophane was used as starting material and was sublimed under vacuum and converted by pyrolysis into the corresponding quinodimethanes, which spontaneously polymerized upon condensation to the cooled substrate surface at 15 °C. Throughout CVD polymerization, a constant argon flow of 20 sccm and a working pressure of 0.3 mbar were maintained. The pyrolysis temperature was set to be 670 °C and sublimation temperatures were between 90 to 110 °C under these conditions. CVD polymerization spontaneously occurred on samples placed on a rotating, cooled sample holder. The resulting poly(4-ethynyl-p-xylylene-co-p-xylylene) film thickness was controlled at 100 nm on all substrates studied. Film thickness was measured using a single wavelength (532 nm) EP3-SW imaging ellipsometry (Nanofilm Technologie GmbH, Germany).

### Dip-pen nanolithography process:

For writing on poly(4-ethynyl-p-xylylene-co-p-xylylene) surfaces, all dip-pen nanolithography was done on a DPN 5000 system (Nanolnk, Skokie, USA). A 1-D cantilever array containing 26 Si₃N₄ tips was first treated with oxygen plasma, 10 sccm, at 100 mTorr and with 30W for 5 min. The tips were then dipped either in Alexa Fluor® 555 azide, triethylammonium salt (150 µg/ml, Invitrogen GmbH, Germany) or biotin azide (PEG4 carboxamide-6-azidohexanyl biotin) (100 µg/ml, Invitrogen GmbH, Germany) solution containing sodium ascorbate (20 mM) and CuSO₄ (10 mM) for 15 min, and then were dried using a nitrogen stream. For writing on poly(4-formyl-p-xylylene-co-p-xylylene) surfaces, a 1-D cantilever array containing 26 Si₃N₄ tips was also treated with oxygen plasma with the same process parameters. The tips were then dipped in biotin-LC-hydrazide (100 µg/ml, Thermo Scientific GmbH, Germany) solution at a pH of 5-6 for 15 min, and then were dried using a nitrogen stream. After DPN writing, the resulting samples were rinsed with DI-water in order to remove loosely bound azide molecules. Finally, the samples were examined using a fluorescence microscope (Eclipse 80i upright microscope, Nikon Instruments, Japan). Prior to incubation of the substrate with streptavidin, the substrate is treated with a blocking agent such as BSA.

In a related method, an azide can be immobilized on the substrate via CVD and a biotin-alkyne bound thereto.

### Direct printing of streptavidin

Direct printing of streptavidin on the substrate can be achieved using streptavidin in a protein carrier matrix, e.g. PEG or glycerol.

### Discussion:

The experiments presented here were carried out with the model system of the cancer cell line MCF-7 and biotinylated anti-EpCAM. Streptavidin spots on the bottom surface function as binding sites. To ensure a saturated binding of anti-EpCAM, 100.000 cells were incubated in 1 mL PBS carrying 0.5 µg biotinylated EpCAM representing approximately 8×10¹² molecules (assuming 45 kDa). Prior incubation experiments have shown that antibody labeling should be carried out in a shaker set to 37 °C for 40 minutes.

Anti-EpCAM is the standard marker for CTC capturing and is therefore used in this study. CTCs derived from a solid tumor express EpCAM as epithelial cells, but they may undergo a transition to a mesenchymal phenotype. This process is known as the epithelial to mesenchymal transition (EMT). Until then, CTCs can be identified and labeled with anti-EpCAM. To the present knowledge, all surface-marker-based CTC capturing approaches focus on EpCAM. This is a severe drawback as EpCAM negative CTCs are invisible for these approaches. By labeling CTCs with other specific surface antibodies, like the human epidermal growth factor receptor 2 (HER-2) in the case of breast cancer, or even with an antibody cocktail, the probability to capture EpCAM negative cells increases. The capturing methods and devices of the present invention are compatible for the application of antibody cocktails without any further adaptation, as shown by no need for adjustment on the microfluidic parameters when switching from EpCAM to other antibodies.

## Claims

1. A method for the immobilization of cells or virus particles of interest expressing one or more predetermined surface marker(s) on defined spots on a solid support, comprising the steps:
(a) providing a biological fluid sample suspected of containing cells or virus particles of interest;
(b) labeling the cells or virus particles of interest with antibodies, antibody fragments or antibody mimetics directed against the one or more predetermined surface marker(s) and carrying a first binding agent; and
(c) contacting the labeled cells or virus particles of interest with a solid support, said solid support comprising an array of defined isolated spots of a solid support-bound second binding agent, wherein the first and second binding agents can bind to each other, and wherein the solid support is part of a microfluidic chamber, said microfluidic chamber comprising:
(i) said solid support; and
(ii) a surface opposing the solid support, said surface having transverse structures that facilitate chaotic mixing of the fluid containing the cells or virus particles of interest when said fluid flows along said surface, wherein said transverse structures are surface ridges having a herringbone-structure.

2. The method according to claim 1, further comprising, after step (c), the step of identifying solid support-bound cells or virus particles of interest microscopically.

3. The method according to claim 1 or claim 2, further comprising, after step (c), the step of isolating one or more individual immobilized cells or virus particles from the solid support.

4. The method according to any one of claims 1 to 3, wherein the biological fluid is selected from the group consisting of blood, amniotic fluid, cerebrospinal fluid, lymphatic fluid, peritoneal fluid, saliva, sputum, urine, and partitions or fractions thereof.

5. The method according to any one of claims 1 to 4, wherein the cells are circulating tumor cells (CTCs).

6. The method according to any one of claims 1 to 5, wherein the surface marker is Epithelial Cell Adhesion Molecule (EpCAM).

7. The method according to any one of claims 1 to 6, wherein the second binding agent is fluorescently labeled.

8. The method according to any one of claims 1 to 7, wherein the first binding agent is biotin and the second binding agent is streptavidin or an analog thereof.

9. The method according to any one of claims 1 to 8, wherein step (c) is performed at a temperature of 28 to 40°C, preferably at 37°C.

10. A device for the isolation of cells or virus particles of interest expressing one or more predetermined surface marker(s), comprising:
(a) a microfluidic chamber comprising:
(i) a solid support comprising an array of defined isolated spots of a solid support-bound binding agent; and
(ii) a surface opposing the solid support, said surface having transverse structures that facilitate chaotic mixing of the fluid containing the cells or virus particles of interest when said fluid flows along said surface, wherein said transverse structures are surface ridges having a herringbone-structure;
and
(b) a capillary micropipette,
wherein the device is configured to carry out the method according to any one of claims 1 to 9.

11. The device according to claim 10, wherein the binding agent is streptavidin or an analog thereof.

12. The device according to claim 10 or claim 11, further comprising means for heating the solid support.

## Patentansprüche

1. Verfahren für die Immobilisierung von Zielzellen oder -viruspartikeln, die einen oder mehrere vorbestimmte Oberflächenmarker exprimieren, an definierten Stellen auf einem festen Träger, umfassend die Schritte:
a) Bereitstellen einer biologischen Fluidprobe, die unter Verdacht steht, Zielzellen oder -viruspartikel zu enthalten;
(b) Markieren der Zielzellen oder -viruspartikel mit Antikörpern, Antikörperfragmenten oder Antikörpermimetika, die gegen den/die einen oder mehreren vorbestimmten Oberflächenmarker gerichtet sind und ein erstes Bindungsmittel tragen; und
(c) Inkontaktbringen der markierten Zielzellen oder -viruspartikeln mit einem festen Träger, wobei der feste Träger eine Anordnung von definierten isolierten Stellen eines an den festen Träger gebundenen zweiten Bindungsmittels umfasst, wobei das erste und zweite Bindungsmittel aneinander binden können, und wobei der feste Träger Teil einer mikrofluidischen Kammer ist, wobei die mikrofluidische Kammer umfasst:
(i) den festen Träger; und
(ii) eine Oberfläche, die dem festen Träger gegenüberliegt, wobei die Oberfläche transversale Strukturen aufweist, die ein chaotisches Mischen des Fluids ermöglichen, das die Zielzellen oder -viruspartikeln enthält, wenn das Fluid entlang der Oberfläche strömt, wobei die transversalen Strukturen Oberflächengrate sind, die eine Fischgrätstruktur aufweisen.

2. Verfahren nach Anspruch 1, weiter umfassend, nach Schritt (c), den Schritt des mikroskopischen Identifizierens von an den festen Träger gebundenen Zielzellen oder -viruspartikeln.

3. Verfahren nach Anspruch 1 oder Anspruch 2, weiter umfassend, nach Schritt (c), den Schritt des Isolierens einer oder mehrerer einzelner immobilisierter Zellen oder Viruspartikel aus dem festen Träger.

4. Verfahren nach einem der Ansprüche 1 bis 3, wobei das biologische Fluid ausgewählt ist aus der Gruppe, bestehend aus Blut, Fruchtwasser, Liquor, Lymphflüssigkeit, Peritonealflüssigkeit, Speichel, Sputum, Urin und Teilen oder Fraktionen davon.

5. Verfahren nach einem der Ansprüche 1 bis 4, wobei die Zellen zirkulierende Tumorzellen (CTCs) sind.

6. Verfahren nach einem der Ansprüche 1 bis 5, wobei der Oberflächenmarker Epitheliales Zelladhäsionsmolekül (EpCAM) ist.

7. Verfahren nach einem der Ansprüche 1 bis 6, wobei das zweite Bindungsmittel fluoreszent markiert ist.

8. Verfahren nach einem der Ansprüche 1 bis 7, wobei das erste Bindungsmittel Biotin und das zweite Bindungsmittel Streptavidin oder ein Analogon davon ist.

9. Verfahren nach einem der Ansprüche 1 bis 8, wobei Schritt (c) bei einer Temperatur von 28 bis 40°C, vorzugsweise bei 37°C, durchgeführt wird.

10. Vorrichtung zur Isolierung von Zielzellen oder -viruspartikeln, die einen oder mehrere vorbestimmte Oberflächenmarker exprimieren, umfassend:
(a) eine mikrofluidische Kammer, umfassend:
(i) einen festen Träger, der eine Anordnung von definierten isolierten Stellen eines an den festen Träger gebundenen Bindungsmittels umfasst; und
(ii) eine Oberfläche, die dem festen Träger gegenüberliegt, wobei die Oberfläche transversale Strukturen aufweist, die ein chaotisches Mischen des Fluids ermöglichen, das die Zielzellen oder -viruspartikeln enthält, wenn das Fluid entlang der Oberfläche strömt, wobei die transversalen Strukturen Oberflächengrate sind, die eine Fischgrätstruktur aufweisen;
und
(b) eine kapillare Mikropipette,
wobei die Vorrichtung ausgelegt ist, das Verfahren nach einem der Ansprüche 1 bis 9 durchzuführen.

11. Vorrichtung nach Anspruch 10, wobei das Bindungsmittel Streptavidin oder ein Analogon davon ist.

12. Vorrichtung nach Anspruch 10 oder Anspruch 11, weiter umfassend Mittel zum Erwärmen des festen Trägers.

## Revendications

1. Procédé d'immobilisation de cellules ou de particules virales d'intérêt exprimant un ou plusieurs marqueurs de surface prédéterminés sur les endroits définis d'un support solide, comprenant les étapes de:
(a) mettre à disposition un échantillon de fluide biologique suspecté de contenir des cellules ou des particules virales d'intérêt;
(b) étiqueter les cellules ou particules virales d'intérêt avec des anticorps, des fragments d'anticorps ou des mimétiques d'anticorps dirigés contre le ou plusieurs marqueurs de surface prédéterminés et porter un premier agent de liaison; et
(c) mettre en contact les cellules ou les particules virales d'intérêt étiquetées avec un support solide, ledit support solide comprenant une matrice d'endroits définis isolés d'un deuxième agent de liaison lié au support solide, dans lequel les premier et deuxième agents de liaison peuvent se lier l'un à l'autre, et dans lequel le support solide fait partie d'une chambre microfluidique, ladite chambre microfluidique comprenant:
(i) ledit support solide; et
(ii) une surface en face du support solide, ladite surface ayant des structures transversales qui facilitent le mélange chaotique du fluide contenant les cellules ou les particules virales d'intérêt quand ledit fluide s'écoule le long de ladite surface, dans lequel lesdites structures transversales sont des bords de surface présentant une structure en chevrons.

2. Procédé selon la revendication 1, comprenant en outre, après l'étape (c), l'étape d'indentification des cellules ou particules virales d'intérêt liées au support solide et ce, de manière microscopique.

3. Procédé selon la revendication 1 ou la revendication 2, comprenant en outre, après l'étape (c), l'étape d'isolation d'une ou de plusieurs cellules ou particules virales individuelles immobilisées du support solide.

4. Procédé selon l'une quelconque des revendications 1 à 3, dans lequel le fluide biologique est sélectionné parmi le groupe consistant en du sang, du fluide amniotique, du fluide cérébrospinal, du fluide lymphatique, du fluide péritonéal, de la salive, du crachat, de l'urine et des parties ou fractions de ceux-ci.

5. Procédé selon l'une quelconque des revendications 1 à 4, dans lequel les cellules sont des cellules tumorales circulantes (CTC).

6. Procédé selon l'une quelconque des revendications 1 à 5, dans lequel le marqueur de surface est la molécule EpCAM (Angl. = *Epithelial Cell Adhesion Molecule*).

7. Procédé selon l'une quelconque des revendications 1 à 6, dans lequel le deuxième agent de liaison est étiqueté par fluorescence.

8. Procédé selon l'une quelconque des revendications 1 à 7, dans lequel le premier agent de liaison est la biotine et le deuxième agent de liaison est la streptavidine ou un analogue de celle-ci.

9. Procédé selon l'une quelconque des revendications 1 à 8, dans lequel l'étape (c) est réalisée à une température allant de 28 à 40°C, de préférence à 37°C.

10. Appareil destiné à l'isolation de cellules ou de particules virales d'intérêt exprimant un ou plusieurs marqueurs de surface prédéterminés, comprenant:
(a) une chambre microfluidique comprenant:
(i) un support solide comprenant une matrice d'endroits isolés définis d'un agent de liaison lié au support solide; et
(ii) une surface en face du support solide, ladite surface ayant des structures transversales qui facilitent le mélange chaotique du fluide contenant les cellules ou les particules virales d'intérêt quand ledit fluide s'écoule le long de ladite surface, dans lequel lesdites structures transversales sont des bords superficiels présentant une structure en chevrons;
et
(b) une micropipette capillaire,
dans lequel l'appareil est configuré pour exécuter le procédé selon l'une quelconque des revendications 1 à 9.

11. Appareil selon la revendication 10, dans lequel l'agent de liaison est la streptavidine ou un analogue de celle-ci.

12. Appareil selon la revendication 10 ou la revendication 11, comprenant en outre un moyen pour chauffer le support solide.
